# EUROPEAN PATENT APPLICATION

(11) **EP 1 972 380 A1**
(43) Date of publication of application: **24.09.2008**
(21) Application number: 08002831.9
(22) Date of filing: 15.02.2008
(51) Int. Cl.: B03D 3/00

(54) **Method for adjusting sedimentation rates of blood or bone marrow**

(30) Priority: 16.02.2007 US 901660 P
(71) Applicant: Harvest Technologies Corporation, Plymouth, Massachusetts 02360 (US)
(72) Inventor: Ellsworth, James R., Plymouth MA 02360 (US); Kevy, Sherwin, Plymouth MA 02360 (US); Jacobsen, May, Plymouth MA 02360 (US)
(74) Representative: Crouch, David John

(57) **Abstract**

One or more chemicals are added to a physiological fluid to alter the density and/or shape of cells in the fluid to provide a desired sedimentation rate of the cells. In a preferred embodiment, the chemical to be added is an anticoagulant, preferably ACD.

## Description

### TECHNICAL FIELD

This invention relates to the fractionation of components of physiological fluids, such as blood, bone marrow, and the like.

### BACKGROUND

It is known to fractionate physiological fluids by gravity separation. Typically this is done with the aid of a centrifuge, which reduces the time required for the gravity separation. In a known system, a processing unit comprises two chambers that are connected for fluid flow between them whereby after centrifugation the lighter components in one chamber may be decanted to the other chamber. Preferably, a dividing element that floats in the physiological fluid is provided in the first chamber to position itself automatically between components to be separated and prevent decant of the heavier components to be retained in the first chamber during decant of the lighter components.

It has proven difficult, however, to design such a dividing element that accommodates physiological fluids having different initial compositions. A particular problem is presented when it is desired to use a processing unit and a centrifuge protocol that have been designed for blood with a different physiological fluid, such as bone marrow aspirate. Another problem is the use of the same centrifuge operating protocol for a sample obtained from a patient having a blood disorder of the type that affects the sedimentation of the various blood components.

In a typical protocol for obtaining platelet rich plasma, for example, whole blood is placed in the first chamber of a processing unit and subjected to centrifugation. After a predetermined period of time, the centrifuge is stopped, and the processing unit is tipped to pour platelet rich plasma into the second chamber, and the platelet rich plasma is subjected to centrifugation once again to separate the platelets from the plasma for further processing. The shape of the floating dividing element and the material from which it is made are chosen on the basis of the sedimentation characteristics of the blood. Thus, the centrifugal forces to which the blood is subjected and the time period of centrifugation are determined (often empirically) to result in the desired separation between red blood cells on the one hand and platelet rich plasma on the other. But, it is usually the case that a protocol such as that, determined for blood, is not effective, for example, to separate bone marrow aspirate into components for recovery of bone marrow stem cells. Thus, it has not been possible in the prior art to provide a single centrifuge protocol and processing unit for processing different physiological fluids, such as blood and bone marrow aspirate.

### SUMMARY OF THE INVENTION

Applicant has discovered that the primary reason for the variance between the results obtained for different fluids is that the cells in the different physiological fluids sediment at different rates. That is, the sedimentation rate of the cells in bone marrow aspirate differs from that of the cells in whole blood, which means that the densities of the several layers in bone marrow aspirate after a given centrifugation protocol arc different from those in blood after application of the same protocol. Because the densities are different in the different physiological fluids, the position of the floating divider will differ, and the composition of the decanted fluids will differ.

There arc various reasons for the different sedimentation rates, but primary ones are the shapes and densities of the cells. Blood is not an ideal fluid but is, instead, a suspension of particles in a fluid. Plasma is generally considered to be a fluid with a density of 1.020, while the density of the red blood cells suspended in the plasma is 1.07 to 1.090, depending on the age of the cells (young red blood cells are less dense) and the physiological characteristics of the person from whom the blood is obtained. A measure of the amount of red blood cells in blood is its hematocrit (Hct), which is defined as the volume of red blood cells per unit volume of blood. Another component of blood is the platelets, and their density is about 1.040. Yet another component is white blood cells, whose density is between that of red blood cells and platelets.

A complication with the foregoing generalities is that physiological fluids are changed by the very act of obtaining them from a donor. For example, lesion of the cells during collection affects the sedimentation rate. A major factor affecting sedimentation is the effect of the various chemicals used during collection on the cells. The chemicals may be absorbed by the cells, which affects their density and may make them larger or make their configuration more round. The chemicals may, alternatively, make the cells release fluids, which makes them shrink and crenate. The cells are, thus, more dense and pack into a smaller volume. Crenated cells also sediment faster than normal cells.

A chemical usually used during collection of physiological fluids is an anticoagulant, and applicant has discovered that anticoagulants have a marked effect on the sedimentation rates of the components of physiological fluids. There are several basic types of anticoagulants. The citrate types, such as ACD and CPD, bind with calcium and prevent coagulation because calcium is required in several areas of the clotting cascade. A second type comprises the heparins, which prevent coagulation by inhibiting the conversion of prothrombin to thrombin. Another type is known as platelet inhibitors, which decrease the platelet function and find most use systemically to prevent or reduce thrombosis. Anticoagulants also assist in the storage of fluids. For example adding dextrose to the anticoagulant provides nutrition to cells and allows longer cell viability during storage in a blood bank. Other anticoagulants, such as EDTA have been developed to facilitate diagnostic testing.

ACD is hypotonic to red blood cells and its effect on collected blood is to cause the red blood cells to pass water through the cell walls into the cell, causing the cell to swell. One result is that the swelled cells are less dense. That is, the volume of a typical red blood cell is 90 femtoliters, its density is 1.080, and its mass is 97.2 femtograms. If the volume of the cell were to increase by thirty percent through the absorption of water (having a density of 1.000), the red blood cell would absorb 27 femtoliters, or 27 fcmtograms, which would make its volume 117 femtoliters and its weight 124.2 femtograms. The density of the red blood cell would then be 1.062. This factor alone clearly affects the final position of the less dense cells in the chamber, and the shape of the swelled cells also alters their rate of sedimentation.

Other chemicals affect the shape and density of the cells. For example NaCI, either solid or in solution, may cause the cell to swell or crenate depending on the concentration. Thus, chemicals such as this may be used in conjunction with the anticoagulant to achieve a desired result.

In one aspect, the invention contemplates the addition of one or more chemicals to a physiological fluid to alter the density and/or shape of cells in the fluid to result in the desired sedimentation rate of the cells. In a preferred embodiment, the chemical to be added is an anticoagulant, preferably ACD.

In another aspect of the invention, a kit comprising a processing chamber and anticoagulants is provided for use with a centrifuge to separate the components of bone marrow aspirate whereby a protocol for the centrifuge designed for another physiological fluid, such as whole blood, may be used.

### BRIEF DESCRIPTION OF THE DRAWINGS

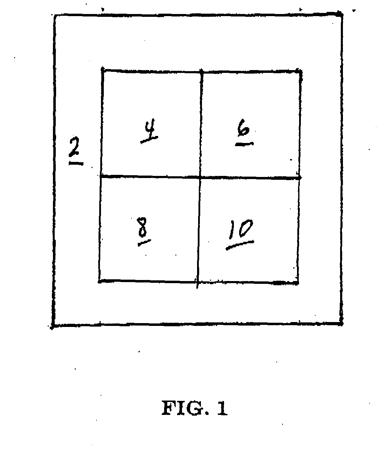

The drawing figure illustrates a kit that may be used in practicing the method of the invention.

### DETAILED DESCRIPTION OF THE PREFERRED EMBODIMENTS

In one specific example of the invention, 60mls of bone marrow aspirate is collected into a bag or syringe having heparin as the anticoagulant. A bag is preferred to be able to inspect the collected marrow. Then, 8-10 mls of ACD are added to the collected aspirate to adjust the sedimentation rate of the components, particularly the red blood cells. The aspirate mixture is then placed in the first chamber of a two-chamber processing unit and subjected to centrifugation according to a known protocol designed to fractionate whole blood for the purpose of making platelet rich plasma as described in USP 6,398,972. The supernatant is then decanted to the second chamber and again subjected to centrifugation according to the said process designed to separate platelets to make platelet rich plasma. Supernatant from the second centrifugation is withdrawn to leave a ten milliliter concentrate sample remaining in the chamber. That sample has been found to be 7.9%Hct, and have a total nucleated cell count (TNC) of 90 x 10³/µl. In contrast, when same process is followed with heparin alone, the sample is 3% Hct with a TNC of 32.4 x 10³/µl.

The above results can be attributed to the modification of the sedimentation rate of cells by the addition of ACD to the aspirate as discussed above. Similar results arc obtained with normal aspirate and aspirate from a patient suffering from a disorder that affects sedimentation.

It has been found that the addition of additional amounts of ACD to whole blood in the production of platelet rich plasma also reduces the difference between concentrate samples obtained from those patients with and those patients without the disorders that affect sedimentation rates. Thus, in a second example, 54mls of whole blood are drawn from these patients into 6mls of ACD as the anticoagulant. An additional 8-10 mls of ACD are then added to reduce the sedimentation rate of red blood cells, and the sample is subjected to the protocol noted above. The results show that the Hct and TNC from concentrate samples do not differ significantly.

The drawing figure is a schematic illustration of a kit 2 in accordance with the invention. The kit includes a syringe 4 (or bag), a processing unit 6, a first container 8 having a prescribed quantity of anticoagulant, and a second container 10 having a prescribed quantity of hypotonic or hypertonic material.

Modifications within the scope of the appended claims will be apparent to those of skill in the art.

## Claims

1. A method for adjusting the sedimentation rate of components of a physiological fluid comprising the step of adding to said physiological fluid an effective amount of a hypotonic or hypertonic material.

2. A method according to claim 1, wherein said physiological fluid is bone marrow aspirate.

3. A method according to claim 1 or claim 2, further comprising the step of adding heparin as an anticoagulant.

4. A method according to any preceding claim, wherein said hypotonic or hypertonic material is hypotonic and comprises ACD.

5. A method according to claim 4 wherein the volume of said physiological fluid is about 54 mls and the volume of said ACD is from about 8 to about 10 mls.

6. A method according to claim 1 wherein said physiological fluid is blood.

7. A method according to claim 6 further comprising the step of adding ACD as an anticoagulant.

8. A method according to claim 6 or claim 7, wherein said hypotonic or hypertonic material is hypotonic and comprises ACD.

9. A method according to claim 8 wherein the volume of said physiological fluid is about 54 mls and the volume of said ACD is from about 8 to about 10 mls.

10. A kit for use in fractionation of bone marrow aspirate comprising a two-chamber processing unit configured to receive a 60 ml sample, a container for withdrawing bone marrow aspirate, heparin for anticoagulation of said bone marrow aspirate, and 8-10 mls ACD for adjusting the sedimentation rate of the components of the aspirate.

11. A kit for use in fractionation of whole blood comprising a two-chamber processing unit configured to receive a 60 ml sample, a syringe for withdrawing whole blood, 6 mls ACD for anticoagulation of said bone marrow aspirate, and 8-10 mls ACD for adjusting the sedimentation rate of the components of the aspirate.
